# EUROPEAN PATENT APPLICATION

(11) **EP 4 610 996 A1**
(43) Date of publication of application: **03.09.2025**
(21) Application number: 23882785.1
(22) Date of filing: 30.10.2023
(51) Int. Cl.: G16H 30/40, A61B 5/00, A61B 6/00

(54) **IMAGE GENERATION DEVICE, IMAGE GENERATION METHOD, DISPLAY DEVICE, IMAGE GENERATION PROGRAM, AND RECORDING MEDIUM**

(30) Priority: 28.10.2022 JP 2022173781
(71) Applicant: KYOCERA Corporation, Kyoto 612-8501 (JP)
(72) Inventor: WATANABE, Kenichi, Kyoto-shi, Kyoto 612-8501 (JP); HONDA, Shintaro, Kyoto-shi, Kyoto 612-8501 (JP); SHIRATORI, Takaaki, Kyoto-shi, Kyoto 612-8501 (JP)
(74) Representative: TBK
(86) International application number: PCT/JP2023/039050
(87) International publication number: WO 2024/090584

(57) **Abstract**

The reliability of an analysis result obtained using a machine model is further improved. An image generation device includes an acquirer that acquires an analysis result output from a machine model that analyzes a medical image obtained by imaging a subject and derivation basis data indicating a basis on which the analysis result is derived, and an image generator that generates a display image obtained by changing the medical image based on the derivation basis data.

## Description

### TECHNICAL FIELD

The present disclosure relates to an image generation device, an image generation method, a display device, an image generation program, and a recording medium.

### BACKGROUND OF INVENTION

For example, Patent Document 1 discloses an X-ray age estimation model that uses data of an X-ray image of an examinee and information on an examinee's age when the X-ray image was captured to indicate a correspondence between a feature quantity obtained from the data of the X-ray image and the examinee's age.

### CITATION LIST

### PATENT LITERATURE

Patent Document 1: JP 2022-148729 A

### SUMMARY

### PROBLEM TO BE SOLVED

In recent years, various images have been analyzed using a trained machine model. However, the basis of an analysis result obtained using the machine model is often unclear. An object of the present disclosure is to further improve the reliability of the analysis result obtained using the machine model.

### SOLUTION TO PROBLEM

In an aspect of the present disclosure, an image generation device includes an acquirer configured to acquire an analysis result output from a machine model configured to analyze a medical image obtained by imaging a subject and derivation basis data indicating a basis on which the analysis result is derived; and an image generator configured to generate a display image obtained by changing the medical image based on the derivation basis data.

In an aspect of the present disclosure, an image generation method includes acquiring an analysis result output from a machine model configured to analyze a medical image obtained by imaging a subject and derivation basis data indicating a basis on which the analysis result is derived; and generating a display image obtained by changing the medical image based on the derivation basis data.

In each aspect of the present disclosure, the image generation device may be implemented by a computer, and in this case, a control program of the image generation device that causes the computer to operate as each unit (software element) of the image generation device to implement the image generation device using the computer, and a computer-readable recording medium on which the control program is recorded are also within the scope of the present disclosure.

### ADVANTAGEOUS EFFECT

According to an aspect of the present disclosure, the reliability of the analysis result obtained using the machine model can be further improved.

### BRIEF DESCRIPTION OF THE DRAWINGS

FIG. 1 is a block diagram illustrating a configuration of an image generation device according to Embodiment 1 of the present disclosure.
FIG. 2 is a flowchart showing a flow of an image display method according to Embodiment 1.
FIG. 3 is a schematic diagram illustrating an example of a display image according to Embodiment 1.
FIG. 4 is a block diagram illustrating a configuration of a remote image analysis system according to Embodiment 2 of the present disclosure.
FIG. 5 is a schematic diagram illustrating an example of a browser image for input by a user.
FIG. 6 is a schematic diagram illustrating an example of X-ray image data for requesting bone density analysis.
FIG. 7 is a schematic diagram illustrating an example of a browser image of an analysis result transmitted by an analysis result transmitter.
FIG. 8 is a schematic diagram illustrating an example of a browser image in which derivation basis data is added to the display image illustrated in FIG. 7.
FIG. 9 is an enlarged schematic diagram of an image of an area illustrated in FIG. 8.
FIG. 10 is a schematic diagram illustrating an example of a browser image displaying a patient's risk of fracture at a present time and in the future.
FIG. 11 is a schematic diagram illustrating an example of a browser image in which derivation basis data has been added to the display image illustrated in FIG. 10.
FIG. 12 is a schematic diagram illustrating an example of a display image in which cytopathological analysis results and derivation basis data thereof have been added.
FIG. 13 is a schematic diagram illustrating an example in which a display image is displayed on a single screen by scrolling.
FIG. 14 is a block diagram illustrating a configuration of a remote image analysis system according to Embodiment 3.
FIG. 15 illustrates an example of an image including derivation basis data of change in the image due to other reasons analyzed by a machine model.
FIG. 16 illustrates an example of a browser image showing an analysis result in both cases including and excluding change in the image due to other reasons.

### DESCRIPTION OF EMBODIMENTS

### Embodiment 1

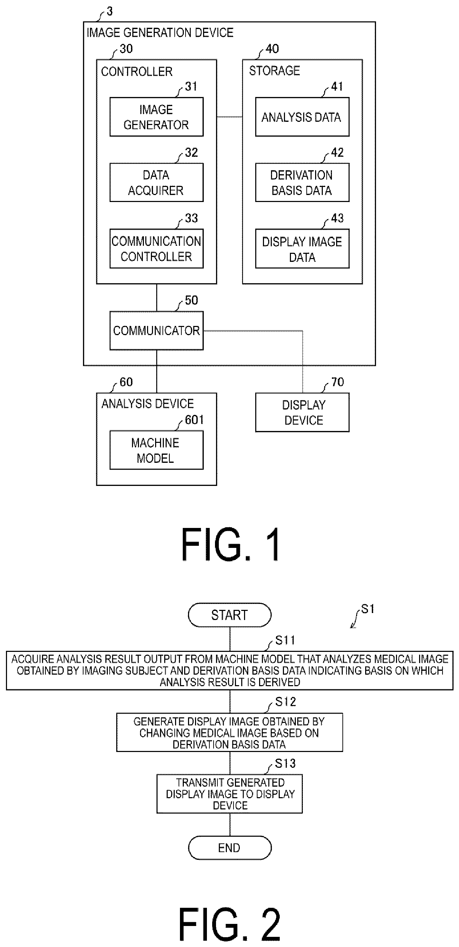

Hereinafter, an embodiment of the present disclosure will be described in detail with reference to the drawings. FIG. 1 is a block diagram illustrating a configuration of an image generation device 3 according to Embodiment 1 of the present disclosure. The image generation device 3 is a device that generates a display image obtained by changing a medical image to include a basis of an analysis result, based on area-of-interest information on an area of interest noted in a process of outputting the analysis result from a machine model that analyzes a medical image. The image generation device 3 is capable of communicating information between an analysis device 60 and a display device 70 via a communicator 50. The analysis device 60 includes a machine model 601. The machine model 601 is, for example, a trained machine model that analyzes an image and/or a numerical value. The display device 70 displays, for example, a result of the analysis in the machine model 601.

As illustrated in FIG. 1, the image generation device 3 includes a controller 30, a storage 40, and a communicator 50. The controller 30 includes an image generator 31, a data acquirer (acquirer) 32, and a communication controller 33.

The data acquirer 32 acquires an analysis result (analysis data) output (derived) from the machine model 601 that analyzes an image, and derivation basis data of the analysis result. The derivation basis data is, for example, area-of-interest information on an area of interest being an area in the medical image and noted in a process of outputting the analysis result. The data acquirer 32 records acquired analysis data 41 and derivation basis data 42 in the storage 40. The medical image may include, for example, at least one selected from the group consisting of a plain X-ray image, a computed tomography (CT) image, a magnetic resonance imaging (MRI) image, a positron emission tomography (PET) image, and an ultrasound image. When the plain X-ray image is used as the medical image, an imaging site of the plain X-ray image is not particularly limited. For example, the plain X-ray image used as the medical image may be an image showing a part of a skeleton of a subject. More specifically, the plain X-ray image may show at least one selected from the group consisting of a head, neck, chest, lumbar region, hip joint, knee joint, ankle joint, foot, toe, shoulder joint, elbow j oint, wrist j oint, hand, finger, and jaw joint of the subject. A plain X-ray image for estimation is used to estimate bone density of bones and a fracture site of the subject. Such a plain X-ray image for estimation may be a front image showing a target site from a front (for example, an image obtained by irradiating the target site with X-rays in a front-back direction) or may be a side image showing the target site from a side (for example, an image obtained by irradiating the target site with X-rays in a left-right direction). When a CT image is used as a medical image, for example, at least one selected from the group consisting of a three-dimensional image, a cross-sectional image (for example, a horizontal section) perpendicular to a body axis connecting a head to a leg, and a cross-sectional image (for example, a sagittal section or a coronal section) parallel to the body axis can be used. The medical image may be an image showing a bone. The medical image may be an image not showing a bone. A bone density of the bone may be expressed by at least one selected from the group consisting of a bone mineral density per unit area (g/cm²), a bone mineral density per unit volume (g/cm³), a YAM (%), a T score, and a Z score. YAM (%) is an abbreviation for "Young Adult Mean" and is sometimes called a young adult average percentage. For example, the bone density of the bone may be a value expressed by bone mineral density per unit area (g/cm²) and YAM (%). The bone density of the bone may be an index determined by a guideline or may be a unique index.

The image generator 31 generates a display image (display image data) obtained by changing the medical image based on the area-of-interest information. The image generator 31 records the generated display image data 43 in the storage 40.

The communication controller 33 transmits the display image data 43 recorded in the storage 40 to the display device 70. In this case, the communication controller 33 may transmit the display image data 43 to the display device 70. For example, the communication controller 33 may first transmit the image data used for analysis, and then transmit the analysis data 41. Alternatively, the communication controller 33 may first transmit the analysis data 41, and then transmit the image data used for analysis.

The controller 30 controls the entire image generation device 3. The controller 30 includes at least one processor and at least one memory. The processor may be configured using a general-purpose processor such as at least one micro processing unit (MPU) or central processing unit (CPU). The memory may include a plurality of types of memory such as a read only memory (ROM) and a random access memory (RAM). As an example, the processor implements a function of the image generation device 3 by loading various control programs recorded in the ROM of the memory into the RAM and executing the programs. The processor may also include a processor configured with an application specific integrated circuit (ASIC), a field programmable gate array (FPGA), a programmable logic device (PLD), or the like.

FIG. 2 is a flowchart showing an example of a flow of an image display method S1 using the image generation device 3. As shown in the figure, the image display method S1 includes steps S11 to S13.

The data acquirer 32 acquires the analysis result output from the machine model 601 that analyzes a medical image obtained by imaging a subject, and derivation basis data indicating the basis on which the analysis result has been derived (S11). Specifically, for example, the data acquirer 32 acquires the analysis result output from the machine model that analyzes a medical image obtained by imaging a subject, and area-of-interest information on an area of interest being an area in the medical image and noted in the process of outputting the analysis result. The area in the medical image may be, for example, an area inside an outer periphery of the medical image. The area-of-interest information may be, for example, located entirely in the area in the medical image, or may overlap only partially. Alternatively, when the area-of-interest information is not present in the medical image (or, for example, when the area-of-interest information is the entire medical image), the area-of-interest information may not overlap with the area in the medical image. **In** this case, for example, a message indicating that the area of interest is not present (or, for example, that the entire medical image is the area of interest) may be displayed outside the area in the medical image. Hereinafter, a case in which the target is a human (that is, a "subject") will be described, but the target is not limited to a human. The target may be, for example, a mammal other than a human, such as those from the equine, feline, canine, bovine, or porcine families. The present disclosure also includes embodiments in which "subject" is reworded as "animal" when the embodiments are applicable to any of these animals.

The image generator 31 generates the display image obtained by changing the medical image based on the acquired derivation basis data (S12). Specifically, the image generator 31 generates the display image obtained by changing the medical image based on, for example, the area-of-interest information.

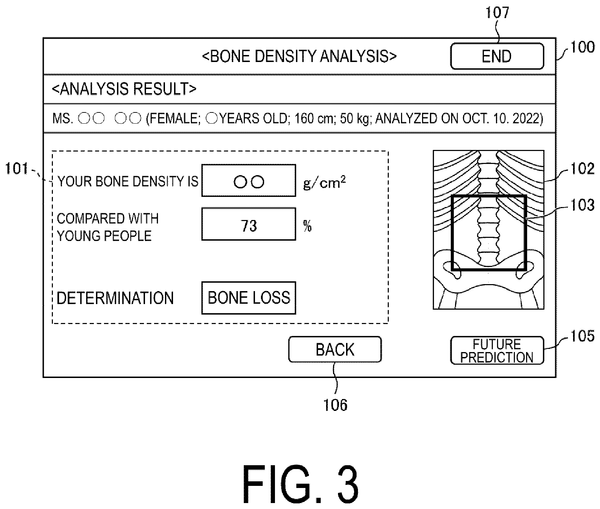

The communication controller 33 transmits the display image generated by the image generator 31 to the display device 70 (step S13). With the above processing method, the analysis result derived by the machine model that analyzes an image and the derivation basis data of the analysis result can be displayed on a single screen. With this method, as an example, as illustrated in FIG. 3, a display image 100 in which the analysis result output from the machine model that analyzes a medical image obtained by imaging the subject and the area-of-interest information on an area of interest being an area in the medical image and noted in the process of outputting the analysis result are displayed on a single screen is generated and displayed on the display device 70.

FIG. 3 shows a schematic diagram illustrating an example of the display image (display screen) 100 according to Embodiment 1. Specifically, FIG. 3 illustrates a display image in which the analysis result derived by the machine model that analyzes an image and derivation basis data of the analysis result are displayed on a single screen. The derivation basis data of the analysis result means at least one basis on which the machine model has derived such an analysis result. For example, the derivation basis data of the analysis result may be information indicating which part of the image (medical image) is mainly used to derive the analysis result, or information indicating which part is weighted to derive the analysis result. The display image is an image displayed on the screen (display screen) of the display device. The display image may be a browser image, that is, an image displayed on a website on the Internet that can be viewed using a browser. The type of display device is not limited. The screen may be, for example, a screen of a stationary personal computer or a screen of a mobile terminal.

In FIG. 3, specifically, the analysis result derived by the machine model 601 that analyzes an image including bones and the derivation basis data of the analysis result are displayed on one display screen (the screen displaying the display image 100). The analysis result is displayed in an area 101, and includes, for example, bone density and a ratio of the bone density to an average bone density of a young adult. The derivation basis data of the analysis result is displayed in an area 102. The image displayed in the area 102 is an image in which an area of interest 103 has been added to an image including bones analyzed by the machine model 601. In the present embodiment, the area of interest is an area that is a main basis for deriving the analysis result of the image. A "Future prediction" button 105, a "Back" button 106, and an "End" button 107 will be described later.

As illustrated in FIG. 3, the analysis result derived by the machine model 601 that analyzes an image and the derivation basis data of the analysis result can be displayed on a single screen. This allows the user to confirm at least one of the bases on which the machine model has derived such an analysis result together with the analysis result.

With the image generation device 3 or the image display method S1 described above, the analysis result derived by the machine model that analyzes an image and the derivation basis data of the analysis result can be displayed on a single screen. This allows the user to confirm one of the bases on which the machine model has derived such an analysis result together with the analysis result. Therefore, the reliability of the analysis result obtained using the machine model can be further improved compared to a case in which only the analysis result is displayed.

### Embodiment 2

Another embodiment of the present disclosure will be described below. In the present embodiment, an example of a display image or the like will be described using a case in which an analysis system (also called a remote image analysis system) 70A that receives an image analysis request from a user analyzes an X-ray image of a bone and outputs bone density, a relative comparison of bone density, a likelihood of fracture (%), and the like. The remote image analysis system 70A can display to the user, one of the bases on which the machine model has derived such an analysis result, together with the analysis result.

### Configuration of Remote Image Analysis System 70A

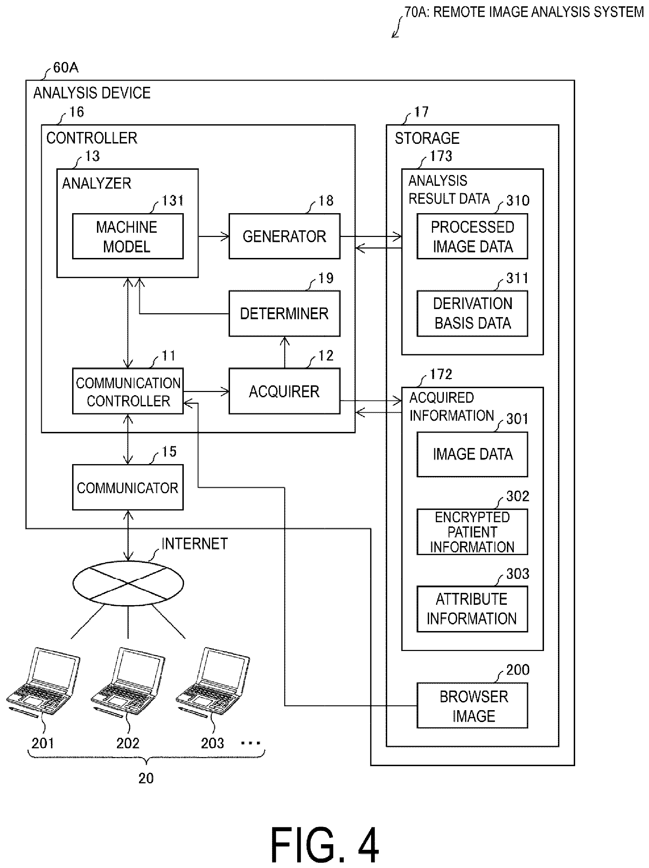

FIG. 4 is a block diagram illustrating a configuration of a remote image analysis system 70A according to Embodiment 2 of the present disclosure. As illustrated in the figure, the remote image analysis system 70A includes an analysis device 60A (image remote analysis device) that analyzes medical data (for example, medical image data) and a user terminal 20, and the analysis device 60A and the user terminal 20 are communicatively connected to each other via the Internet.

The analysis device 60A includes a communicator 15, a controller 16, and a storage 17. The communicator 15 communicates with the user terminal 20 via the Internet. The storage 17 stores acquired information 172 including image data 301, encrypted patient information 302, and attribute information 303, and analysis result data 173 including processed image data 310 and derivation basis data 311. The storage 17 stores the image data 301, the encrypted patient information 302, and the attribute information 303 acquired by the acquirer 12 in association with each other.

The controller 16 includes a communication controller 11 (analysis result transmitter), an acquirer 12, an analyzer 13, a generator (image generator) 18, and a determiner 19. The communication controller 11 controls the communicator 15. The acquirer 12 acquires data transmitted from the user terminal 20 via the communication controller 11. The acquirer 12 also acquires an analysis result (analysis data) derived by the analyzer 13 (machine model 131) that analyzes a medical image, and derivation basis data of the analysis result. The analyzer 13 analyzes the image data 301. The controller 16 may have the same or similar configuration as the controller 30 described in Embodiment 1.

The generator 18 generates the derivation basis data of the analysis result analyzed by the analyzer 13. The derivation basis data 311 means at least one basis on which the machine model has derived such an analysis result. For example, the derivation basis data 311 may be information indicating which part of the image is mainly used to derive the analysis result. The derivation basis data 311 is linked to at least the encrypted patient information 302 and stored in the storage 17. A specific example of a function of the generator 18 will be described later. The generator 18 generates a display image (display image data) in which the analysis result acquired by the acquirer 12 and the area-of-interest information (derivation basis data of the analysis result) are displayed on a single screen. The generator 18 records the generated display image data in the storage 17.

### Determiner 19

The determiner 19 determines whether the encrypted patient information 302 acquired by the acquirer 12 is information obtained by actually encrypting the patient identification information 320. When it is determined that the encrypted patient information 302 is not actually encrypted, the acquirer 12 may delete the acquired encrypted patient information 302. By providing such a determiner 19, if the encrypted patient information 302 transmitted from the user terminal 20 is not information obtained by encrypting the patient identification information 320, the acquired image data 301 is deleted together with the encrypted patient information 302 and the attribute information 303 without being analyzed. Therefore, the acquired information 172 including the image data 301 is not stored inside the analysis device 60A. Whether data has been encrypted may be determined from, for example, an extension of the encrypted data.

When it is determined that the encrypted patient information 302 is not actually encrypted, the controller 16 may send a message for requesting to encrypt and send the patient identification information 320 again to the user terminal 20 via the communication controller 11.

### Browser Image 200

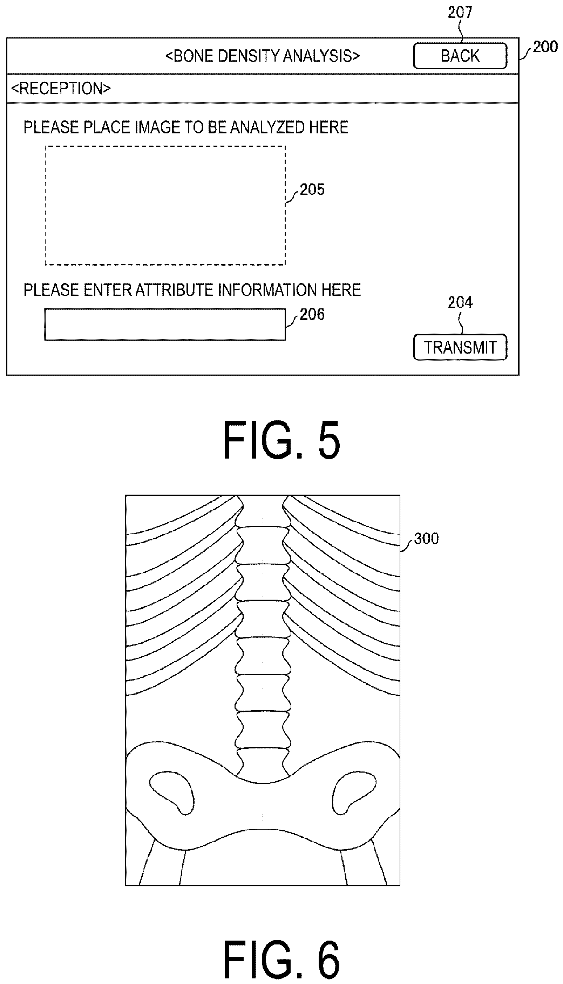

FIG. 5 is a schematic diagram illustrating an example of the browser image 200 displayed on a web page to input the image data 301 or the like when a user accesses the analysis device 60A via the Internet.

As shown in the figure, text "bone density analysis" indicating a target of image analysis is displayed at the top of the browser image 200. The text displayed at the top is not limited thereto, and may be, for example, a display indicating analysis content, that is, "Bone density estimation" and "Future bone density prediction". At an upper left corner of the browser image 200, situation information indicating a phase of the analysis or the content of the analysis on a current screen may be described. For example, <Reception>, <Analysis result>, <Future prediction>, <Benign/malignant determination>, and the like at the upper left corner of the browser image 200 may be used, but the present disclosure is not limited thereto.

For example, the word "Reception" indicating that a screen is to receive an input is displayed at the upper left corner of the browser image 200. Below that, the text "Place the image to be analyzed here" to prompt the user to input image data to be analyzed, and a frame 205 indicating an area where image data is pasted (drag and drop) are displayed. Further, below that, the text "Input attribute information here" to prompt the user to input the patient identification information 320 and the attribute information 303, and a box 206 for input is displayed. The "Transmit" button 204 for prompting transmission is displayed at the lower right part of the browser image 200, and the "Back" button 207 for returning to the initial screen of the website is displayed at the upper right part.

FIG. 6 is a schematic diagram illustrating X-ray image data 300 for requesting bone density analysis as an example of the image data 301. The X-ray image data 300 may be a plain X-ray image such as a lumbar X-ray image or a chest X-ray image, or may be an X-ray image captured with a DXA (Dual energy X-ray Absorptiometry) device or the like. In the DXA device that measures bone density using a DXA method, when the bone density of a lumbar spine is measured, X-rays are radiated from a front of the lumbar spine of the subject. In the DXA apparatus, when the bone density of the proximal femur is measured, X-rays are emitted to the proximal femur of the subject from the front of the proximal femur. An ultrasound method is a method of measuring bone density by applying ultrasound to bones of a heel, shin, or the like. Here, "the front of the lumbar spine" and "the front of the femur" are intended to be directions that correctly face an imaging site such as the lumbar spine and the femur, and may be the ventral side of a subject's body or the back side of a subject. In the micro densitometry (MD) method, x-rays are emitted to hands. An ultrasonic method is a method of measuring bone density by applying ultrasonic waves to a bone such as the lumbar vertebrae, femur, heel, or shank. The image data need not be an X-ray image but may be an image including information of bones. The image data may be estimated from, for example, a magnetic resonance imaging (MRI) image, a computed tomography (CT) image, a PET image, or an ultrasound image.

In the present embodiment, when the user accesses the analysis device 60A via the Internet, the communication controller 11 may display the browser image 200 on a web page and transmit an encrypted application via the communicator 15. When the user inputs the image data 301, the patient identification information 320, and the attribute information 303 to be requested to be analyzed and clicks the "Transmit" button 204, the patient identification information 320 is encrypted, and the encrypted patient information 302 is transmitted to the analysis device 60A together with the image data 301 and the attribute information 303. Therefore, the user does not need to perform encryption processing of the patient identification information 320. The encryption process need not be performed when the user clicks the "Transmit" button 204, but may be performed after the user clicks the "Transmit" button.

The encryption scheme may be a known scheme and is not limited thereto. For example, the encryption scheme may be an encryption scheme in which a public key is combined with a private key. The encryption may be a method that cannot be decrypted even by an operator of the remote image analysis system 70A. This reduces the risk that the combination of the image data 301 and the patient identification information 320 will be leaked to the operator of the remote image analysis system 70A even when the user requests image analysis from the operator of the remote image analysis system 70A.

The acquirer 12 acquires the X-ray image data 300, the encrypted patient information 302, and the attribute information 303 transmitted from the user terminal 20 and transmits the X-ray image data 300, the encrypted patient information 302, and the attribute information 303 to the analyzer 13.

The analyzer 13 inputs the X-ray image data 300 transmitted from the acquirer 12 to the machine model 131, processes the output data from the machine model 131 as necessary, and generates the analysis result data 173. The generated analysis result data 173 is associated with at least the encrypted patient information 302 and the attribute information 303, and stored in the storage 17.

The communication controller 11 acquires the analysis result data 173 linked to the encrypted patient information 302 and the attribute information 303 from the storage 17, and transmits the analysis result data 173 to the user terminal 20 via the communicator 15.

When the user terminal 20 receives the analysis result data 173, the encrypted patient information 302 is automatically decrypted and the patient identification information 320 is generated. The encrypted patient information 302 may be decrypted when the analysis result data 173 is transmitted, or may be decrypted by the user himself/herself.

As a result, the user terminal 20 can display the analysis result data 173 on the screen together with the patient identification information 320 including the patient's name or identification number.

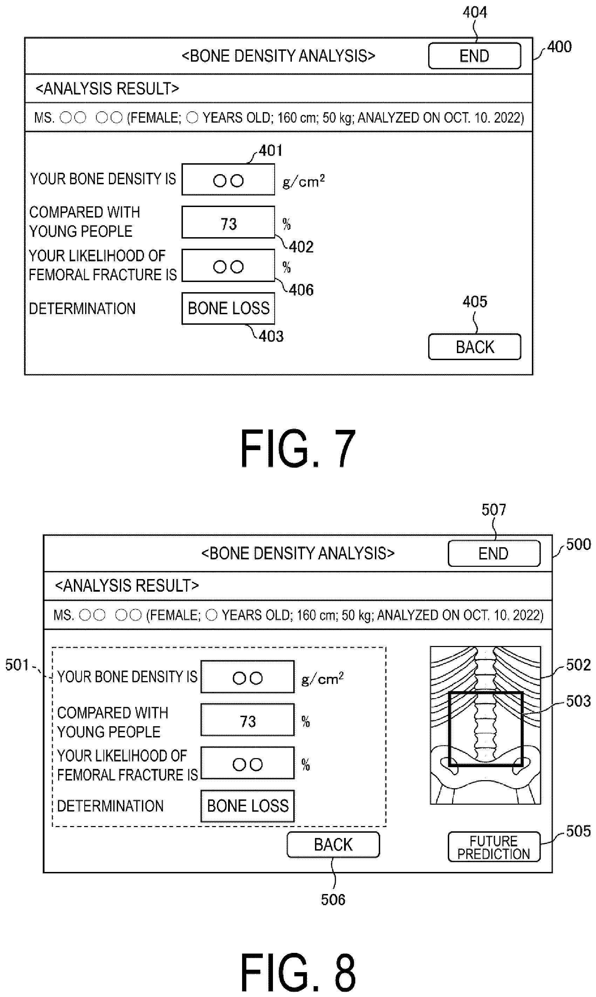

FIG. 7 is a schematic diagram illustrating an example of a browser image 400 displayed on a screen of the user terminal 20 that has received the analysis result data 173. The browser image 400 is an example of the display image data generated by the generator 18. The text "bone density analysis", which indicates the content of the image analysis, is displayed at the top of the browser image 400. In the upper left part of the browser image 400, the text "Analysis Result" indicating that the screen is displaying the analysis result is displayed. The patient information may be displayed below that. Below that, the text "Your bone density is □g/cm²" is displayed. In box 401, the estimated bone density value is displayed. Further, the text "□% of the bone density of young people" may be displayed below it. In a box 402, the average bone density in young adults, that is, the Young Adult Mean (YAM) is displayed. Below that, the text "Your femur fracture likelihood is □%" may be displayed. In a box 406, the fracture likelihood is displayed in %. Below that, the text "Determination" may be displayed, and the text "Bone mass reduction" may be displayed in a box 403. For example, if the percentage to YAM is less than 80%, the patient is determined to have "bone loss", and if the percentage is 70% or less, the patient is determined to maybe have "osteoporosis". The browser image 400 may include an "End" button 404 and a "Back" button 405. The "Back" button may be, for example, a button to return to the previous screen, may be a button to return to a home screen, or may be a button to return to a specific screen.

### Generator 18

The generator 18 will be described in detail. In the present embodiment, the generator 18 generates derivation basis data 311 of an analysis result obtained from analysis by the analyzer 13. The communication controller 11 may transmit the derivation basis data 311 generated by the generator 18 to the user terminal 20.

The analysis result data 173 is based on the output from the machine model 131, but the output from the machine model 131 does not include an analysis process. Thus, generally, the reliability of the output cannot be determined only by looking at the output from the machine model 131. Therefore, transmitting the analysis result data 173 including the derivation basis data 311 to the user terminal 20 can help improve the user's reliability in the analysis result.

For example, the derivation basis data 311 may be processed image data 310 (basis image data) in which new information is added to the image data 301 input to the machine model 131. For example, the new information may be information indicating an area serving as a main basis for deriving an analysis result in the image data 301. The processed image may be an image in which the information indicating the area that is the main basis is added to the input image data. The information indicating the area is, for example, information indicating a range of the area, such as coloring or framing. In image analysis, there are many cases in which a certain area of an image serves as a main basis for estimation, and thus, the user can confirm the area serving as the basis for estimation based on information indicating such an area. In this case, the display image displayed on the user terminal 20 includes a processed image in which the area-of-interest information is added to the medical image input to the machine model 131. The generator 18 may generate a display image in which the medical image (image data 301) and the processed image are displayed in parallel. Such a display image makes it easy for the user to compare the original image that has been analyzed with the processed image. Alternatively, the controller 16 may switch between the medical image and the processed image and display the image according to a user operation. For example, the controller 16 may alternately display the medical image and the processed image by the user clicking a switch button.

The processed image data 310 does not need to include all pieces of information of the image data 301 input by the user. For example, the processed image data 310 may be image data obtained by trimming the input image data 301, or may be an image having a resolution lower than that of the input image data 301. In this way, the amount of data to be transmitted and received can be reduced.

FIG. 8 is a schematic diagram illustrating an example of a browser image 500 that transmits the derivation basis data 311 to the user terminal 20 in addition to the analysis result data 173. The browser image 500 is an example of the display image data generated by the generator 18. In the browser image 500, the derivation basis data 311 (a processed image data 502 including an area 503) is added to the analysis result data 173 illustrated in FIG. 7.

Specifically, in the browser image 500, the processed image data 502 obtained by additionally processing a rectangular area 503 to the input image data (X-ray image data 300 in FIG. 6) is displayed together with the analysis result 501. As illustrated in FIG. 8, in the present embodiment, the analysis result 501 derived by the machine model 131 that analyzes the image data and the processed image data 502 including the derivation basis data 311 are displayed on a single screen. In the browser image 500, a "Back" button 506, an "End" button 507, and a "Future prediction" button 505 may be displayed. The role of the "Future prediction" button 505 will be described below.

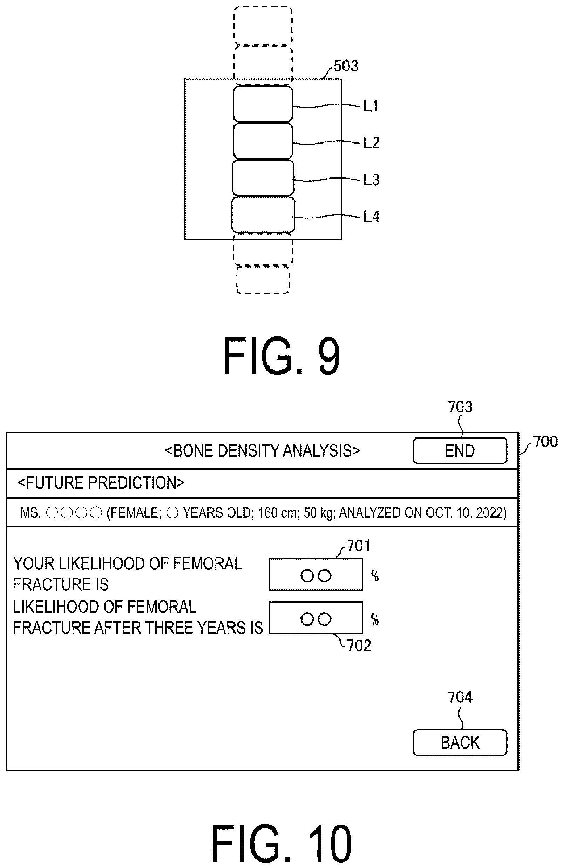

FIG. 9 is an enlarged schematic diagram of the image of the area 503 in FIG. 8. The area 503 includes four lumbar vertebrae indicated by L1 to L4. This indicates that the lumbar vertebrae L1 to L4 are the areas serving as the basis of the analysis result. Actually, it is known that the bone density of lumbar vertebrae L1 to L4 is related to the mean value of the bone density of the whole body. That is, the framed area 503 indicates that the analysis result of the machine model 131 is derived based on the bone densities of the lumbar vertebrae L1 to L4.

The area of interest serving as the basis of the analysis result may include an area obtained by segmenting the medical image. The area of interest serving as the basis of the analysis result may include at least a part of an area obtained by segmenting the medical image. The area of interest serving as the basis of the analysis result may include an entire area obtained by segmenting the medical image. Segmentation is dividing an image into several areas. Segmentation is performed to reduce the amount of the analysis process of the machine model 131. That is, the machine model 131 may analyze only the segmented areas. The segmented areas may be set to an arbitrary range. Each segmented area may be, for example, a rectangle, a square, or a circle. When the segmented area is a square, the amount of analysis process of the machine model 131 can be reduced. When the X-ray image of the lumbar vertebra is analyzed, for example, the range including the lumbar vertebrae L1 to L4 is segmented. Therefore, the size of the segmented areas may change depending on the size of the lumbar vertebrae L1 to L4 in the image. In the arrangement direction of the lumbar vertebrae L1 to L4, for example, the segmented areas may be set to be slightly larger than the lumbar vertebrae L1 to L4, or may be set to overlap the sides of the lumbar vertebrae L1 to L4. For example, the segmented areas may always have a predetermined size, or may be set to have a size according to the medical image. For example, the segmented areas may be set by specifying the positions of the lumbar vertebrae L1 to 4, setting the lengths of the lumbar vertebrae L1 to L4 in the arrangement direction, and then setting the lengths of the lumbar vertebrae L1 to L4 in the vertical direction. The segmentation may be performed by the machine model 131. The machine model 131 may have learned an annotated image of the analysis area in order to perform segmentation.

The generator 18 may generate a heat map of the area serving as the basis of the analysis result. In this case, for example, the outer edge of the heat map indicates the segmented areas. A heat map is a method of representing the magnitude of bone density by the concentration of an arbitrary color. For example, the generator 18 may generate a heat map indicating the degree of focus. A heat map indicating numerical values of bone density may be generated. The generator 18 may generate a heat map indicating likelihood (probability) of fracture. The processed image may be an image in which a heat map showing the area-of-interest information is superimposed on the medical image. The image used for the heat map may be a still image or a video. By representing a heat map with a moving image, for example, by fading various heat maps in order, the relationship between heat maps can be visually recognized with ease. When an analysis result is a heat map of bone density including areas other than the segmented areas, a part of the segmented areas may be surrounded by a frame.

The generator 18 may acquire information of the area serving as the basis of the analysis result from the analyzer 13. Specifically, the generator 18 may acquire the area serving as the basis of the analysis result from the analyzer 13 and generate information indicating the area (a frame line surrounding the area 503 or the like). The generator 18 may acquire the degree of focus data, the bone density data, or the information indicating the likelihood of fracture in the area from the analyzer 13 and generate a heat map. The generator 18 may generate any two or more of a heat map indicating the degree of focus data, a heat map indicating the bone density data, and a heat map indicating the likelihood of fracture. When the heat map indicating the degree of focus data, the heat map indicating the bone density data, and the heat map indicating the likelihood of fracture are displayed in an overlapping manner, the generator 18 may make the color of the heat map indicating the degree of focus data, the color of the heat map indicating the bone density data, and the color of the heat map indicating the likelihood of fracture different from each other.

The machine model 131 analyzes, for example, the X-ray image data 300 using a neural network model (NNM). In the NNM, the image is divided into small areas, each of which is quantified, and the plurality of areas are pooled to be integrated into a larger area and quantified again, and this process is repeated. Therefore, the machine model 131 may extract an area having a numerical value (for example, a relatively large numerical value) that affects the processing result as an area serving as a basis.

In the example illustrated in FIG. 8, the processed image data 502 has been described in which the area 503 serving as the basis of the analysis result is superimposed on the X-ray image data 300 that is an analysis target.
However, the processed image data is not limited thereto. For example, the analysis device 60A may transmit position information (such as coordinates) of the area serving as the basis of the analysis result in an image that is an analysis target to the user terminal 20, and cause the user terminal 20 to display the processed image data in which the area is displayed on the image that is an analysis target.

The screen illustrated in FIG. 8 can be used when a doctor explains the analysis result to a patient. That is, it is possible not only to explain the analysis result to the patient but also to explain the area of the image data that served as the basis for the analysis result. Therefore, not only can the reliability of the doctor with respect to the analysis result be improved but also an effect that the patient is easily satisfied with the analysis result is obtained.

In the example illustrated in FIG. 8, the analysis result 501 and the processed image data 502 including the derivation basis data 311 are displayed on a single screen.
However, "the single screen" may not be simultaneously displayed on the screen. The analysis result 501 and the processed image data 502 may be displayed by scrolling the screen up and down or left and right, for example. In other words, in the analysis result 501 and the processed image data 502, a range displayed by scrolling the screen up and down or left and right is referred to as "the single screen".

FIG. 10 is a schematic diagram illustrating an example of a browser image 700 that displays a bone site where a fracture is expected at a present time of a patient estimated from the image data, a likelihood of the fracture at that site, and a likelihood of the fracture at that site of the patient three years from now, which are analyzed by the analyzer 13. The future time is not limited to three years from now, and may be any time (for example, X years from now). The browser image 700 is an example of the display image data generated by the generator 18. This is displayed by clicking a "Future prediction" button 505 at the bottom right of the browser image 500 illustrated in FIG. 8. The prediction is not limited to the future, and may be a prediction of a time different from a point in time when the medical image has been captured. Specifically, the time may be a time before the point in time when the medical image has been captured, and in this case, a "past prediction" can be displayed instead of or in addition to the "future prediction".

In the browser image 700, the text "bone density analysis" indicating a target of the image analysis is displayed at the top.
In the upper left part of the browser image 700, the text "Future prediction" indicating that the screen displays future prediction is displayed. The patient information may be displayed below that. At the lower part, the text "Your likelihood of femoral fracture is □%" is displayed. In a box 701, a numerical value of a femur fracture likelihood estimated at the present time is displayed. Further, below that, the text "Your femur fracture likelihood in 3 years is □%" may be displayed. A box 702 displays the numerical value of the predicted likelihood of fracture after three years. Information on the estimated basis or the predicted basis may be displayed together with the image.

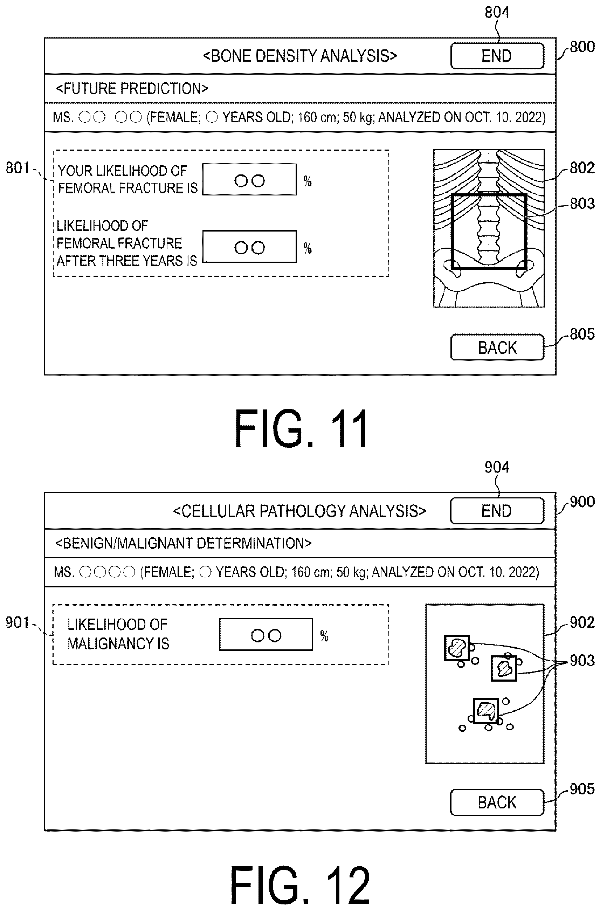

FIG. 11 is a diagram illustrating an example of a browser image 800 in which derivation basis data is added to the display image illustrated in FIG. 10. The browser image 800 is an example of the display image data generated by the generator 18. As illustrated in FIG. 11, in the browser image 800, an image 802 showing derivation basis data is displayed in addition to an area 801 displaying the femur fracture likelihood at the present time and in 3 years, which is derived by the machine model 131.

Such a browser image 800 can be used when a doctor explains to a patient the likelihood of the fracture at the present time and in the future. When information about the estimation basis or prediction basis is also displayed, an effect of increasing persuasiveness to the patient can be obtained.

### Machine Model 131

The machine model 131 will be described. The machine model 131 is a model that estimates a state of a bone of a subject, input image data is an image including bone, and the machine model 131 outputs an estimation result related to the bone condition as the analysis result. For example, the machine model 131 is a trained model that has been trained to output an estimation result or a calculation result related to the bone condition, such as bone density, a relative comparison of bone density, and a likelihood of fracture, from an X-ray image of a bone. The bone density may be a calculated bone density of a bone part included in the image data, or an average bone density of a whole body estimated from the image data. As a method of calculating a bone density from an image, a known method can be used. The relative comparison of bone densities is the ratio of estimated bone density with respect to YAM. The likelihood of fracture is a likelihood that a bone in a specific part (for example, a femoral neck) will fracture. An estimation result regarding a state of a bone may be an estimation result at the time when an image was captured or may be prediction at the time after a predetermined period elapsed from the aforementioned time.

The machine model 131 may output, as an estimation result, at least one selected from the group consisting of bone density estimated at a point in time when the image data is captured, bone density predicted at a point in time when a predetermined period has elapsed since the image data is captured, a fracture site and its likelihood estimated at a point in time when the image data is captured, and a fracture site and its likelihood predicted at a point in time when a predetermined period has elapsed since the image data is captured.

### Training Method for Machine Model 131

The method of training the machine model 131 will be described. Training of the machine model 131, for example, training of estimating a bone density may be performed using an X-ray image of a bone whose bone density has been specified as teacher data. Training of estimating likelihood of a fracture may be performed by using an X-ray image of a bone and data indicating whether the patient suffered fracture within a predetermined period thereafter as teacher data. The relative comparison of bone densities does not have to be a subject for training and is obtained by dividing the estimated bone density by the YAM. Training of future prediction may be performed using an X-ray image of a bone whose bone density has been specified and data indicating the bone density of the patient after a predetermined period of time thereafter or whether the patient has suffered fracture as teacher data. The machine model 131 capable of performing more accurate estimation or prediction by including, in the teacher data, lifestyles such as an exercise amount, types of food for meals, smoking, and drinking of the patient serving as the teacher data.

In Embodiment 2, the machine model 131 has been described as an example of a machine model that has been trained to estimate the condition of bones, such as bone density. In this case, the input image data is an X-ray image of the bone, and the output is an estimation result regarding the state of the bone. However, the machine model 131 is not limited to such a model. For example, the machine model 131 may be a cytopathology analysis model. In this case, the input medical image may be a microscopic image of subject's cells, and the analysis result may be information on pathological mutations in the cells.

FIG. 12 is a schematic diagram illustrating an example of a browser image 900, which is a display image to which a cytopathological analysis result and its derivation basis data are added when the machine model 131 is a cytopathological analysis model. The browser image 900 is an example of the display image data generated by the generator 18. In the browser image 900 illustrated in FIG. 12, text "cytopathological analysis" indicating the content of the image analysis is displayed at the top. In an upper left corner of the browser image 900, the text "determination result" indicating that the screen displays a result of determining whether the cell is benign or malignant is displayed. The patient information may be displayed below that.
In an area 901 below that, text "The likelihood of malignancy is □%" are displayed. In the box, a numerical value of a likelihood that the cell is malignant is displayed. Further, an image 902 in which an area 903 which is a main basis for deriving a determination result is added to the analyzed image is displayed to the right of the area 901 as the derivation basis data. The area 903 is an area including cells determined to be malignant by the machine model 131. An "End" button 904 and a "Back" button 905 may be displayed in the browser image 900.

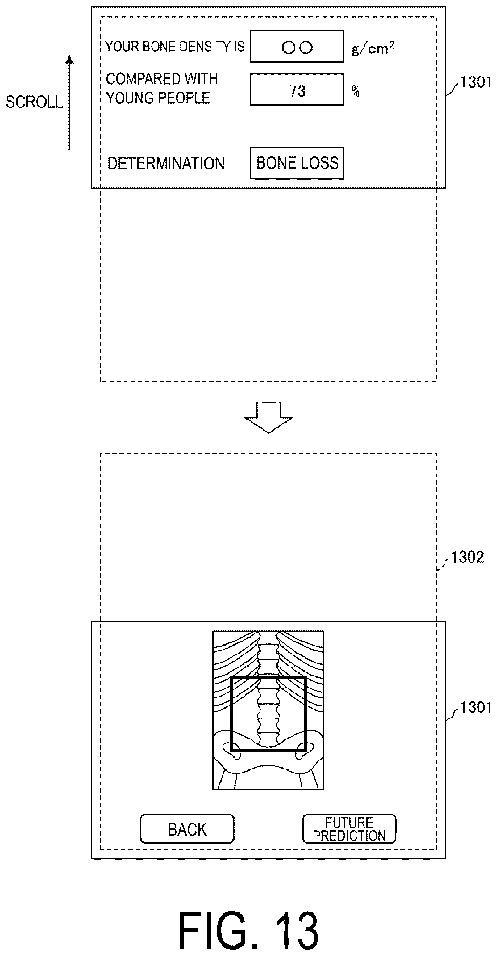

The single screen may be a screen displayed by screen scrolling. FIG. 13 is a schematic diagram illustrating an example of displaying a display image on a single screen by scrolling. An area 1301 shown by a solid line in FIG. 13 is a display area of the user terminal 20 (display device). An area 1302 shown by a dotted line is a single screen. In an upper part of the area 1302, data of a bone density analysis result 501 described in FIG. 8 is displayed. In a lower part of the area 1302, the processed image data 502, which is the basis derivation data, described in FIG. 8, is displayed. When the area 1302 is first displayed in the area 1301, the data of the bone density analysis result 501 is displayed as shown in an upper part of FIG. 13. However, when the user scrolls the area 1302 up, the processed image data 502 appears as shown in the upper part of FIG. 13. Even with this configuration, the user can display the analysis result and its derivation basis data simply by scrolling the screen.

According to a configuration of the remote image analysis system 70A according to Embodiment 2, the derivation basis data 311 can be provided to the user together with the analysis result data 173. This has the effect of improving the user's reliability in the analysis result. Further, the effect of making the patient more likely to understand the analysis result is obtained when the analysis result is explained to the patient.

A flow of the image remote analysis method S2 executed by the controller 16 according to Embodiment 2 will be described. The image remote analysis method S2 includes steps S21 to S24 (not shown).

When the user accesses the analysis device 60A, the communication controller 11 displays on the web page via the communicator 15 the browser image 200, which is an input screen for inputting the acquired information 172 including the image data 301, the patient identification information 320, and the attribute information 303 of the analysis target (S21).

The acquirer 12 acquires encrypted patient information 302 in which the image data 301, the attribute information 303, and the patient identification information 320 included in the acquired information 172 input to the browser image 200 and transmitted from the user terminal 20 have been encrypted (S22).

The analyzer 13 analyzes the image data 301 transmitted from the acquirer 12 using the machine model 131 (S23).

The communication controller 11 transmits the analysis result data 173 analyzed by the analyzer 13, the derivation basis data 311, and the encrypted patient information 302 to the user terminal 20 (S24).

According to the image remote analysis method S2, the derivation basis data 311 can be provided to the user together with the analysis result data 173. This has the effect of improving the user's reliability in the analysis result. Further, the effect of making the patient more likely to understand the analysis result is obtained when the analysis result is explained to the patient.

### Embodiment 3

Another embodiment of the present disclosure will be described below. In Embodiment 2, an analysis system has been described that analyzes the X-ray images of the patient's bones and outputs the bone density, the relative comparison of bone density, the likelihood of fracture (%), and the like. However, in an image (for example, the plain X-ray image) in which at least a portion of the bone is shown, change in the image due to other reasons may appear in addition to change due to a decrease in bone density. The other reasons include some diseases, treatment scars, various types of intentionally input information, and items worn by the patient. Some diseases are, for example, calcification of an artery, hardening of the bone, fracture, and tumor in an organ. The treatment scars are, for example, implants and bone cements. Intentionally input information is, for example, letters such as "L" and "R" that indicate a direction. Items worn by the patient are, for example, a necklace, and the like.

Due to such causes, the brightness (whiteness) of the image often changes compared to when there is no other reason. In a case of implants, necklaces, and the like, unique shapes appear. Such changes affect the evaluation of the bone density based on the analysis in the analyzer 13 of the analysis device 60A of the remote image analysis system 70A described in Embodiment 2. Usually, a doctor who sees the image notices such changes and interprets the evaluation result of the bone density after taking the information that caused such changes into account. However, in some cases, the doctor may not notice such changes in the image. Alternatively, the doctor may not be able to determine to what extent the analysis result of the analyzer 13 takes change in the image due to other reasons into account. In such a case, the doctor may be confused about how to interpret the analysis result of the analyzer 13.

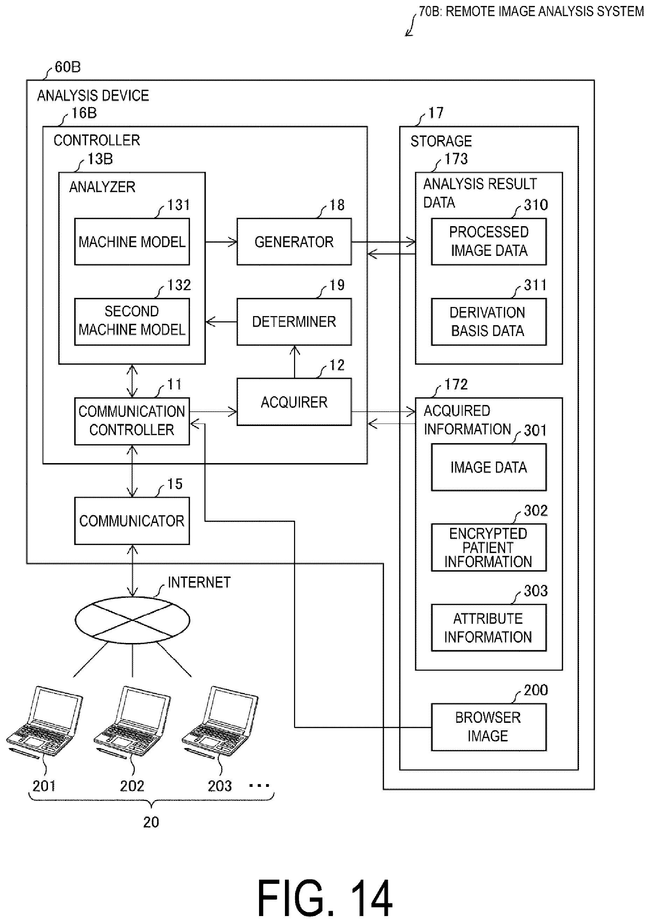

Therefore, it is desirable for the machine model to be able to analyze not only a target object itself but also the change in the image due to other reasons. The remote image analysis system 70B according to the present embodiment can execute at least one of two analysis processing operations when the machine model performs an analysis related to the state of the target object (for example, bones) itself in the image, detects change in the image due to other reasons, that is, reasons different than the state of the bone, and performs an analysis including the changes, and when the machine model performs an analysis excluding the changes. FIG. 14 is a block diagram illustrating a configuration of a remote image analysis system 70B according to Embodiment 3. The same as or similar to the remote image analysis system 70A according to Embodiment 2, the remote image analysis system 70B includes an analysis device 60B (image remote analysis device) that analyzes medical data (for example, medical image data) and a user terminal 20, and the analysis device 60B and the user terminal 20 are communicatively connected to each other via the Internet.

The analysis device 60B includes a communicator 15, a controller 16B, and a storage 17. The communicator 15 and the storage 17 have the same or similar functions as the communicator 15 and the storage 17 described in Embodiment 2, and therefore description thereof will be omitted here. The controller 16B includes a communication controller 11, an acquirer 12, an analyzer 13B, a generator (image generator) 18, and a determiner 19. The components of the controller 16B, except for the analyzer 13B, have the same or similar functions as the components described in Embodiment 2, and therefore description thereof will be omitted here.

The analyzer 13B includes a machine model 131 and a second machine model 132. The machine model 131 is a machine model that performs an analysis of a state of the target object (for example, bones), for example, as in Embodiment 2. On the other hand, the second machine model 132 is a machine model that has been trained to detect the change in the image due to other reasons, such as changes in the image due to some disease, treatment scars, various types of intentionally input information, items worn by the patient, and the like. The second machine model 132 can be trained using images in which the doctor has annotated positions of the image due to some diseases, treatment scars, various types of intentionally input information, items worn by the patient, and the like. The machine model 131 can analyze the state of the target object including the change in the image due to other reasons detected by the second machine model 132. Further, the machine model 131 can analyze the state of the target object excluding the change in the image due to other reasons detected by the second machine model 132.

The analyzer 13B includes one machine model, and the machine model may perform analysis regarding a state of the target object (for example, bones) itself, and detect change in the image due to other reasons to execute two analysis processing operations of a case of analysis including the change and a case of analysis excluding the change.

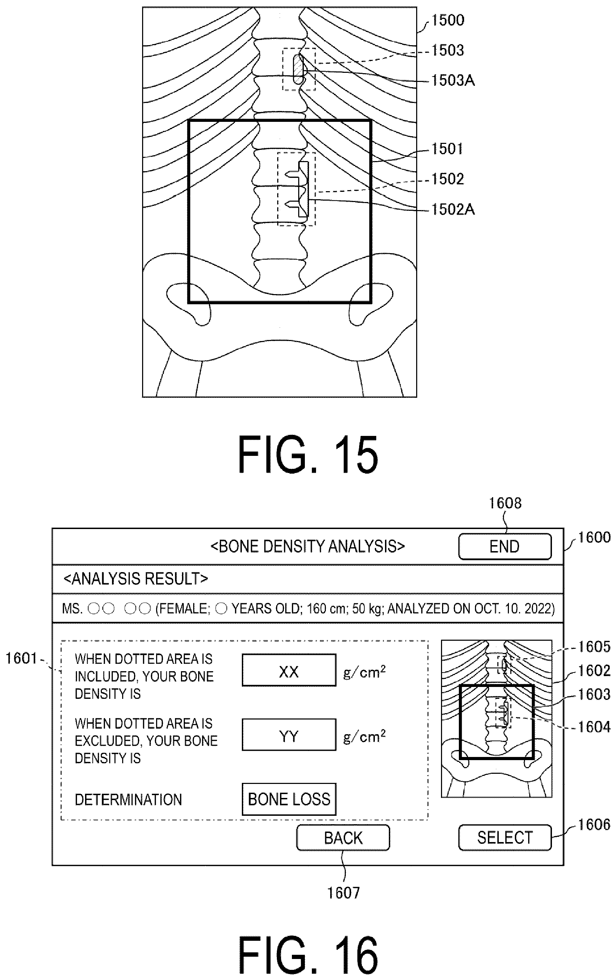

FIG. 15 illustrates an example of an image including derivation basis data of change in the image due to other reasons, generated by the generator 18 based on the analysis result of the second machine model 132. An area indicated by a frame 1502 in FIG. 15 indicates an area in which an implant for fixing the spine is reflected. An area indicated by a frame 1503 indicates an area in which a calcified abdominal aorta is reflected. In the figure, each area is indicated by a dotted line, but may be indicated by different colors, such as a red frame and a yellow frame. An area indicated by a frame 1501 indicates an area that is the derivation basis of the analysis result of the bone density of the lumbar of the machine model 131, as in Embodiment 2. Thus, a range in which the change in the image due to other reasons is detected may be outside the area indicated by the frame 1501 that is the derivation basis when an analysis of the state of the target object is performed.

The analyzer 13B may perform an analysis of the state of the bone, including change in the image due to other reasons. The analyzer 13B may perform the analysis of the state of the bone excluding change in the image due to other reasons. The generator 18 may generate analysis results for both a case in which the change in the image due to other reasons is included and a case in which the change in the image due to other reasons is excluded. The user may designate which analysis method to use and which analysis result to display. When a plurality of change locations are present, the user may designate which analysis method to use and which analysis result to display for each of the change locations.

FIG. 16 is an example of a browser image 1600 that displays analysis results for both a case in which the change in the image due to other reasons is included and a case in which the change in the image due to other reasons is excluded. In this browser image 1600, for example, an analysis image 1602 and an analysis result 1601 are displayed. The analysis image 1602 is the same as or similar to the image illustrated in FIG. 15. In the analysis result 1601, "When a dotted line area is included, your bone density is XX g/cm²" and "when the dotted line area is excluded, your bone density is YY g/cm²" are displayed. Below the analysis image 1602, a selection button 1606 is present, and when this is clicked, the screen can be moved to a screen where a selection whether to include one or both of the dotted line areas 1604 and 1605 in the analysis, which analysis result to display, and the like can be performed. A back button 1607 and an end button 1608 are the same as before.

An example in which the remote image analysis system 70B evaluates the bone density of the femur or vertebra has been described above. However, the target object to be evaluated is not limited thereto. For example, the remote image analysis system 70B may evaluate an X-ray image of a chest bone, or may analyze an image of a target object other than the bones.

As described above, according to the remote image analysis system 70B of the present embodiment, as illustrated in FIG. 15, for example, the area indicated by the frame 1501 serving as the basis for deriving the analysis result regarding the state of the bone, and the area indicated by the frame 1502 and 1503 that are the basis for deriving the change in the image due to other reasons can be displayed in one image. As illustrated in FIG. 16, whether the result of the analysis includes change in the image due to other reasons or the result of the analysis excludes change in the image due to other reasons can be displayed. With this configuration, the doctor can confirm under what conditions the analysis result of the image output by the remote image analysis system 70B has been obtained. Therefore, an interpretation of the analysis result of the image output by the remote image analysis system 70B can be accurately performed.

### Example of Implementation using Software

The functions of the remote image analysis system 70A (hereinafter referred to as a "system") can be implemented by a program for causing a computer to serve as the system, which is a program for causing the computer to serve as each unit of the system.

In this case, each of the systems includes a computer having at least one control device (e.g., processor) and at least one storage device (e.g., memory) as hardware for executing the program. By executing the program by the control device and the storage device, the functions described in the embodiment are implemented.

The program may be recorded on one or more computer-readable non-transitory recording media. This recording media may be or need not be included in the device. In the latter case, the program may be supplied to the apparatus via any wired or wireless transmission medium.

Some or all of the functions of each of the above-described units can be implemented by logic circuits. For example, an integrated circuit in which logic circuits functioning as each of the above-described units is also included in the scope of the present disclosure. In addition to this, for example, a quantum computer can implement the functions of each of the above-described units.

The invention according to the present disclosure has been described above based on the drawings and examples. However, the invention according to the present disclosure is not limited to each embodiment described above. That is, the embodiments of the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, note that a person skilled in the art can easily make different variations or modifications based on the present disclosure. Note that these variations or modifications are within the scope of the present disclosure.

In the present disclosure, a case in which the medical image is analyzed and the medical image is changed based on the area-of-interest information on an area of interest noted in a process of outputting the analysis result has been described, but the present disclosure is not limited thereto. For example, analysis may be performed using a machine model that has been trained with only numerical values or the following information data as input information without including a medical image. In this case, element of interest information noted in the process of outputting the analysis result may be displayed on the display image together with the analysis result. The element of interest information may also be information in which a part of the input information is highlighted. For example, when information of age and sex is an element of interest, items or numerical values of the age and sex may be highlighted in a conspicuous color.

The medical image may be, for example, an image of a subject photographed with an endoscope. More specifically, the medical image includes an image obtained by photographing a site including at least one selected from the group consisting of a nasal cavity, an esophagus, a stomach, a duodenum, a rectum, a large intestine, a small intestine, an anus, and a colon of the subject with an endoscope. The medical image obtained by imaging these sites may output the analysis result that clearly indicates the site of interest including at least one selected from the group consisting of inflammation, polyps, and cancer using a learning model. In such a case, the learning model may be a model trained based on, for example, a first learning image including an image with a site of interest, first teacher data indicating the presence of the site of interest, a second learning image including an image without a site of interest, and second teacher data indicating the absence of the site of interest. The first teacher data may include information indicating an inflammation level (degree of inflammation) or malignancy (degree of malignancy) of the site of interest. The analysis result may be, for example, a display surrounding the site of interest, a display indicating the site of interest, or a display in which a color is superimposed on the site of interest. Along with such an analysis result, the derivation basis data indicating the basis on which the analysis result has been derived may be displayed in the same or similar manner as in the above-described embodiment.

Alternatively, the medical image may be, for example, an image obtained by photographing eyes, skin, and the like of the subject with a digital camera. The medical image obtained by imaging these sites may output, for example, an analysis result indicating a sign of interest using the learning model. The signs of interest may include, for example, signs indicating diseases including at least one selected from the group consisting of glaucoma, cataracts, age-related macular degeneration, conjunctivitis, hordeolum, retinopathy, and blepharitis, in the case of eyes. Alternatively, the sign of interest may include, for example, signs including skin cancer, urticaria, atopic dermatitis, and herpes in the case of skin. The analysis result may be displayed by surrounding these signs of interest, by pointing to the signs of interest, by superimposing a color on the signs of interest, or by displaying a name of the disease. As the learning model, for example, a model trained based on the first learning image having an image including these sites of interest, first teacher data indicating the presence of the signs of interest, a second learning image including an image without the signs of interest, and second teacher data indicating the absence of the signs of interest may be used. Along with such an analysis result, the derivation basis data indicating the basis on which the analysis result has been derived may be displayed in the same or similar manner as in the above-described embodiment.

The input information may include age, sex, weight, height, presence or absence of fracture, fracture location, fracture history, family (for example, parents') fracture history, underlying diseases (for example, food and/or drug allergies, diseases related to onset of osteoporosis, and diseases unrelated thereto), smoking history, drinking habits (for example, drinking frequency and amount), occupational history, exercise history, medical history (for example, history of bone disease), menstruation (for example, cycle and presence or absence), menopause (for example, likelihood and presence or absence), artificial joint (for example, type, presence or absence of spinal implant or knee joint, and timing of replacement surgery), blood test results, urine test results, medications being taken, and gene sequence.

### Conclusion

### Aspect 1

An image generation device according to aspect 1 of the present disclosure includes an acquirer configured to acquire an analysis result output from a machine model configured to analyze a medical image obtained by imaging a subject and area-of-interest information on an area of interest being an area in the medical image and noted in a process of outputting the analysis result; and an image generator configured to generate a display image obtained by changing the medical image based on the area-of-interest information.

### Aspect 2

The image generation device according to aspect 2 of the present disclosure in which, in aspect 1, the display image includes a processed image in which the area-of-interest information is added to the medical image input to the machine model.

### Aspect 3

The image generation device according to aspect 3 of the present disclosure in which, in aspect 1 or 2, an area of interest includes an area in which the medical image is segmented.

### Aspect 4

The image generation device according to aspect 4 of the present disclosure in which, in any one of aspects 1 to 3, the area-of-interest information is indicated in a form of a heat map.

### Aspect 5

The image generation device according to aspect 5 of the present disclosure in which, in aspect 2, the processed image is an image on which the area-of-interest information is superimposed.

### Aspect 6

The image generation device according to aspect 6 of the present disclosure in which, in aspect 2 or 5, the image generator generates the display image in which the medical image and the processed image are arranged in parallel.

### Aspect 7

The image generation device according to aspect 7 of the present disclosure in which, in any one of aspects 2, 5, and 6, the display image and the processed image are interchangeable according to an operation.

### Aspect 8

The image generation device according to aspect 8 of the present disclosure in which, in any one of aspects 1 to 7, the machine model is a model configured to make an estimation regarding a state of bones of the subject, the medical image is an image showing bones of the subject, and the analysis result includes an estimation result regarding a state of the bones of the subject.

### Aspect 9

The image generation device according to aspect 9 of the present disclosure in which, in any one of aspects 1 to 8, in the display image, the analysis result and the area-of-interest information are indicated in a form of different aspects of heat maps.

### Aspect 10

The image generation device according to aspect 10 of the present disclosure in which, in aspect 8, the estimation result is a result of estimating a fracture site of the subject and/or a likelihood that the subject will have a fracture.

### Aspect 11

The image generation device according to aspect 11 of the present disclosure in which, in any one of aspects 1 to 7, the machine model is a cytopathological analysis model, the medical image is a microscopic image obtained by imaging subject's cells, and the analysis result includes information on pathological mutations in the subject's cells.

### Aspect 12

The image generation device according to aspect 12 of the present disclosure in which, in any one of aspects 1 to 11, the display image is a display image to be displayed on a single screen.

### Aspect 13

The image generation device according to aspect 13 of the present disclosure in which, in aspect 1, the machine model detects change in an image due to reasons other than a state of a target object, and executes at least one of two analysis processing operations of analysis including the change and analysis excluding the change.

### Aspect 14

The image generation device according to aspect 14 of the present disclosure in which, in aspect 13, the machine model includes a machine model configured to perform estimation regarding the state of a target object itself, and a second machine model configured to detect change in the image due to reasons other than the state of the target object.

### Aspect 15

The image generation device according to aspect 15 of the present disclosure in which, in aspect 13 or 14, the image generation device is configured to display a result of the analysis including the change in the image and/or a result of the analysis excluding the change.

### Aspect 16

An image generation method according to aspect 16 of the present disclosure includes acquiring an analysis result output from a machine model configured to analyze a medical image obtained by imaging a subject and area-of-interest information on an area of interest being an area in the medical image and noted in a process of outputting the analysis result; and generating a display image obtained by changing the medical image based on the area-of-interest information.

### Aspect 17

A program according to aspect 17 of the present disclosure is an image generation program configured to cause a computer to serve as the image generation device according to aspect 1, the image generation program causing the computer to serve as the acquirer and the image generator.

### Aspect 18

A recording medium according to aspect 18 of the present disclosure is a computer-readable non-transitory recording medium on which the image generation program according to claim 17 is recorded.

### Aspect 19

A display device according to aspect 19 of the present disclosure displays an analysis result output from a machine model configured to analyze a medical image obtained by imaging a subject, and a processed image obtained by processing the medical image based on area-of-interest information on an area of interest being an area in the medical image and noted in a process of outputting the analysis result.

### REFERENCE SIGNS

- 70A, 70B: Remote image analysis system
- 3: Image generation device
- 11, 33: Communication controller
- 12: Acquirer
- 13: Analyzer
- 131: Machine model
- 14: Analysis result transmitter
- 15, 50: Communicator
- 16, 30: Controller
- 17, 40: Storage
- 18: Generator
- 19: Determiner
- 20: User terminal
- 31: Image generator
- 32: Data acquirer (acquirer)
- 41: Analysis data
- 42: Derivation basis data
- 43: Display image data
- 60, 60A, 60B: Analysis device
- 601: Machine model
- 70: Display device

## Claims

1. An image generation device comprising:
an acquirer configured to acquire an analysis result output from a machine model configured to analyze a medical image obtained by imaging a subject and derivation basis data indicating a basis on which the analysis result is derived; and
an image generator configured to generate a display image obtained by changing the medical image based on the derivation basis data.

2. The image generation device according to claim 1, wherein the display image comprises a processed image in which the derivation basis data is added to the medical image input to the machine model.

3. The image generation device according to claim 1 or 2, wherein an area of interest serving as the derivation basis data comprises an area in which the medical image is segmented.

4. The image generation device according to any one of claims 1 to 3, wherein the derivation basis data is indicated in a form of a heat map.

5. The image generation device according to claim 2, wherein the processed image is an image on which the derivation basis data is superimposed.

6. The image generation device according to claim 2, wherein the image generator generates the display image in which the medical image and the processed image are arranged in parallel.

7. The image generation device according to claim 2, wherein the medical image and the processed image are interchangeable according to an operation.

8. The image generation device according to any one of claims 1 to 7, wherein
the machine model is a model configured to make an estimation regarding a state of bones of the subject,
the medical image is an image showing bones of the subject, and
the analysis result comprises an estimation result regarding a state of the bones of the subject.

9. The image generation device according to claim 8, wherein, in the display image, the analysis result and the derivation basis data are indicated in a form of different aspects of heat maps.

10. The image generation device according to claim 8 or 9, wherein the estimation result is a result of estimating a fracture site of the subject and/or a likelihood that the subject will have a fracture.

11. The image generation device according to any one of claims 1 to 7, wherein
the machine model is a cytopathological analysis model,
the medical image is a microscopic image obtained by imaging a subject's cells, and
the analysis result comprises information on pathological mutations in the subject's cells.

12. The image generation device according to any one of claims 1 to 11, wherein the display image is a display image to be displayed on a single screen.

13. The image generation device according to any one of claims 1 to 12, wherein the machine model detects change in an image due to reasons other than a state of a target object, and executes at least one of two analysis processing operations of analysis comprising the change and analysis excluding the change.

14. The image generation device according to claim 13, wherein the machine model comprises a machine model configured to perform estimation regarding the state of a target object itself, and a second machine model configured to detect change in the image due to reasons other than the state of the target object.

15. The image generation device according to claim 13 or 14, wherein the image generation device is configured to display a result of the analysis comprising the change in the image and/or a result of the analysis excluding the change.

16. An image generation method comprising:
acquiring an analysis result output from a machine model configured to analyze a medical image obtained by imaging a subject and derivation basis data indicating a basis on which the analysis result is derived; and
generating a display image obtained by changing the medical image based on the derivation basis data.

17. An image generation program configured to cause a computer to serve as the image generation device according to claim 1, the image generation program causing the computer to serve as the acquirer and the image generator.

18. A computer-readable non-transitory recording medium on which the image generation program according to claim 17 is recorded.

19. A display device configured to display
an analysis result output from a machine model configured to analyze a medical image obtained by imaging a subject, and
a processed image obtained by processing the medical image based on area-of-interest information on an area of interest being an area in the medical image and noted in a process of outputting the analysis result.
